# EUROPEAN PATENT APPLICATION

(11) **EP 3 505 178 A1**
(43) Date of publication of application: **03.07.2019**
(21) Application number: 17306970.9
(22) Date of filing: 29.12.2017
(51) Int. Cl.: A61K 38/17, A61L 27/52, A61K 31/395, A61K 31/712, C07H 19/04, C07H 19/20, C12N 5/071, B33Y 80/00, A61K 47/54, C08G 83/00, C08J 3/075

(54) **BIOINK GEL FORMULATIONS COMPRISING NUCLEOLIPID-BASED COMPOUND**

(71) Applicant: UNIVERSITE DE BORDEAUX, 33000 Bordeaux (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE, 75016 Paris (FR); Centre Hospitalier Universitaire de Bordeaux, 33400 Talence (FR)
(72) Inventor: BARTHÉLÉMY, Philippe, 33700 MÉRIGNAC (FR); CRAUSTE-MANCIET, Sylvie, 33000 BORDEAUX (FR); DEVILLARD, Raphaël, 33200 BORDEAUX (FR); DESSANE, Bérangère, 33000 BORDEAUX (FR); SMIRANI, Rawen, 33100 BORDEAUX (FR)
(74) Representative: Novagraaf Technologies

(57) **Abstract**

The present invention relates to nucleolipid-based compounds for use in regenerative medicine. More specifically, the invention relates to the use of to nucleolipid-based compounds in bioink gel formulations and a bioprinting method comprising the use of a bioink formulation comprising said nucleolipid-based compounds. The invention further relates to a bioprinted construct comprising nucleolipid-based compound(s) and bioink gel formulations.

## Description

### Technical field

The present invention relates to nucleolipid-based compounds for use in regenerative medicine. More specifically, the invention relates to the use of nucleolipid-based compounds in bioink gel formulations and to a bioprinting method comprising the use of a bioink formulation comprising said nucleolipid-based compounds. The invention further relates to a bioprinted construct comprising nucleolipid-based compound(s) and bioink gel formulations.

In the following description, references between brackets (**[]**) direct to the list of references presented at the end of the description.

### State of the art

Three-dimensional (3D) printing is a growing field in regenerative medicine and represents a valuable tool for the construction of complex 3D structures **[1,2].**

In literature, two approaches involving 3D printing are described: first, the use of 3D printing to build an acellular scaffold which is seeded with cells after fabrication (Top-Down approach), and secondly **[3]**, the creation of tissue constructs by directly printing a biological ink or bioink composed of a mixture of cells and biomaterial (Bottom-Up approach). This latter method can be performed through several techniques as: inkjet, laser and extrusion-based bioprintings **[4]**.

Regarding these different methods, extrusion-based bioprinting (EBB) is part of the most widespread tools used in additive manufacturing, mainly due to its capabilities for building large volume constructs such as tissue or organ equivalents **[3,5]**. This technique offers a large opportunity in terms of bioink as it can be adaptable to a wide range of materials including scaffold-based (hydrogels, microcarriers, decellularized matrix components) and scaffold-free (cells aggregates) bioinks **[6]**. Murphy et al. **[1]** exposed their concept of "ideal material" which needs to respond to several features: printability, biocompatibility, degradation kinetics and byproducts, structural and mechanical properties, material biomimicry.

Among all types of bioinks, hydrogels seem to be a promising biomaterial as they are easy to handle, affordable, and can mimic extracellular matrix **[7]**. Hydrogels are mainly composed of water entrapped in a 3D network of molecules, which can be covalently (chemically) or physically (ionic, hydrogens bonds or hydrophobic effect) crosslinked **[6,7]**.

Initial approaches of extrusion based bioprinting used synthetic polymers such as polyethylene glycol (PEG) or methacrylated gelatin (GeIMA) as scaffold materials but they suffer from poor biomimicry as their synthetic nature does not provide them a biological activity for their use in cell culture **[8]**. As an alternative, natural polymers derived from fibrous proteins or peptides have been developed for their original biological activities.

Among natural polymers, alginate, chitosan, collagen and gelatin are actually the most studied despite their weaknesses. Indeed, these polymers encounter several issues such as low mechanical properties [3], the need of an external crosslinker to form a solid-like structure e.g. reticulation of alginate with calcium cation **[9]** and variability of collagen samples properties from a batch to another **[10]**. Moreover, a prerequisite for a bioink is the capacity of the supramolecular hydrogel to be formulated in a cell culture medium, as for cell viability **[8**,**11]**.

Physically-crosslinked hydrogels, also known as supramolecular hydrogels **[12]**, represent a promising tool in biological applications as their physical properties vary reversibly with conditions or stimuli (temperature, etc.). For this reason, physical hydrogels formed by low molecular weight hydrogelators (LMWHs) **[13**,**14]** developed a great interest in biomedical applications due to their self-assembly abilities and biocompatibility **[15]**. Among LMWHs, the bioinspired amphiphilic compounds such as nucleotide lipid (NL) and glycosyl nucleoside lipid (GNL) display promising properties due to their chemical structure and their resulting physico-chemical properties **[16-19]**. Composed of natural moieties like nucleoside/nucleotide, sugar and aliphatic chain, these low molecular systems allow a better biocompatibility, biostability and biodegradability. Their self-assembling properties in water to form hydrogels demonstrated enhanced rheological properties particularly thixotropy. In addition to their bioinspired structures which favour cell compatibility; these properties highlighted them as promising candidates for EBB.

But not all of bioinspired amphiphilic compounds display suitable properties to be incorporated in bioinks.

There is therefore a need for a hydrogel that does not only provide a biological activity for its use in cell culture but also high mechanical properties.

### Description of the invention

Applicants have thus successfully developed a bioink formulation that fulfils both the need of a biological activity and high mechanical properties.

The invention relates to a use of a nucleolipid-based compound in a bioink formulation.

The invention further relates to the use of a nucleolipid-based compound of formula I: 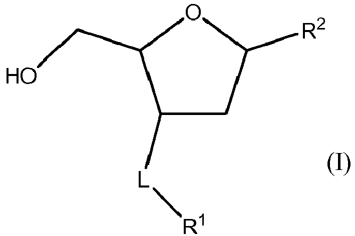 wherein,
- L represents an oxycarbonyl -O-C(O)- group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₂-C₃₀ hydrocarbon chain,
- R¹ represents
   o a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether, or
   o a diacylglycerol (or diglyceride) in which each acyl chain is a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether,
- R² represents a purine or pyrimidine base selected from uracil, adenine, guanine, cytosine, thymine, hypoxanthine, or their derivatives, optionally substituted, or a non-natural mono- or bicyclic heterocyclic base each ring of which comprising from 4 to 7 members, preferably guanine, cytosine or thymine and more preferably thymine, and
the compound being optionally in the form of a salt, preferably a metal cation salt and more preferably a metal cation selected from Li⁺, Na⁺, K⁺, Rb⁺ and Cs⁺,
in a bioink formulation.

In the context of the invention, it is meant by "non-natural heterocyclic base", a base different from uracil, adenine, guanine, cytosine, thymine or hypoxanthine and that cannot be found in nature.

It is meant by "derivative", a compound that is derived from a similar compound by a chemical reaction.

Advantageously, the purine or pyrimidine base, or the non-natural heterocyclic base can be substituted by at least one substituent chosen, for example, from a group comprising a halogen, an amino, a carboxy, a carbonyl, a carbonylamino, a hydroxy, an azido, a cyano, an alkyl, a cyclo alkyl, a perfluoroalkyl, an alkoxy, an oxycarbonyl, a vinyl, an ethynyl, a propynyl or an acyl group.

It is meant by "heteroaryl", a monocyclic or bicyclic, aromatic or partially unsaturated, carbocyclic group containing 5 to 12 atoms, interrupted by 1 to 4 nitrogen atoms, in particular a pyrazole, triazole, tetrazole or imidazole group.

It is meant by "diacylglycerol" or "diglyceride", a glyceride consisting of two acid chains covalently bonded to a glycerol molecule through ester linkages. Two possible forms exist, 1,2-diacylglycerols (formula A) and 1,3-diacylglycerols (formula B):

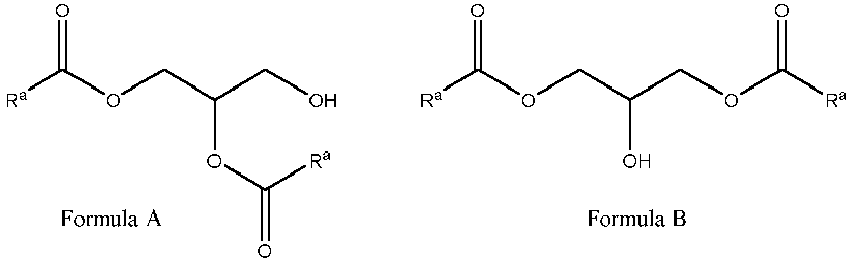

Advantageously, the nucleolipid-based compound may be of formula II: 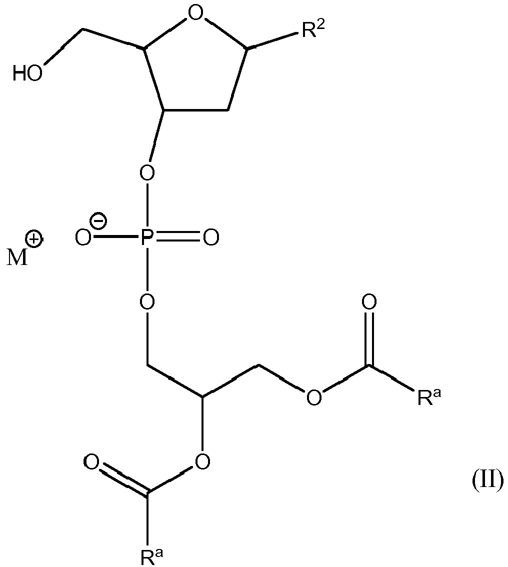 wherein,
- each R^{a}, identical or different, is a linear or branched C₁ to C₂₉ hydrocarbon chain, preferably C₅ to C₂₄ and more preferably C₇ to C₂₄, saturated or unsaturated,
- R² represents a purine or pyrimidine base selected from uracil, adenine, guanine, cytosine, thymine, hypoxanthine, or their derivatives, optionally substituted, preferably guanine, cytosine or thymine and more preferably thymine, and

M⁺ represents a cation, preferably a metal cation, and more preferably selected from Li⁺, Na⁺, K⁺, Rb⁺, Cs⁺.

Advantageously, R^{a}, identical or different, may be a C₁₃ to C₁₉ linear hydrocarbon chain, preferably a C₁₅ linear hydrocarbon chain.

Advantageously, the nucleolipid-based compound may be of formula III: 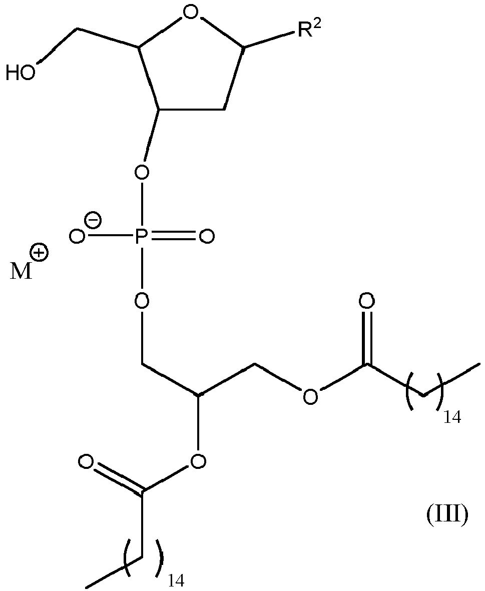 wherein R² represents a purine or pyrimidine base selected from guanine, cytosine and thymine, preferably R² represents a pyrimidine base and more preferably R² represents thymine and M⁺ is as defined above.

Advantageously, the nucleolipid-based compound may be selected from compounds of formula IIIa, IIIb and IIIc, preferably of formula IIIa: 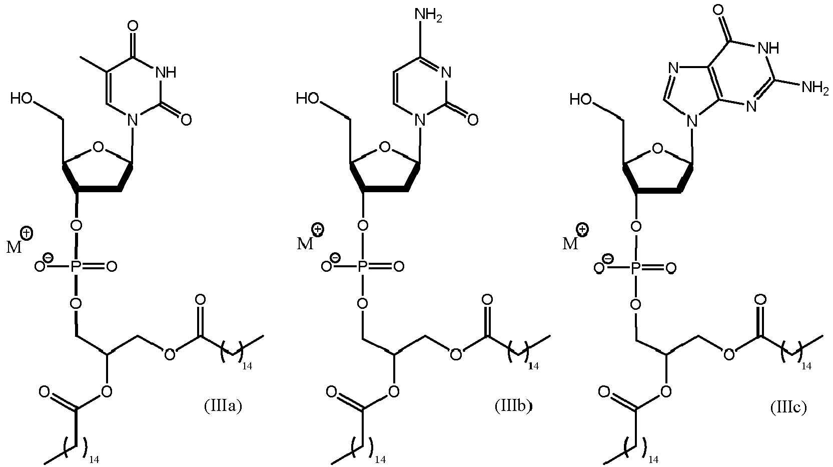 M⁺ being as defined above.

"Bioprinting or "3D bioprinting" is the process of creating cell patterns in a confined space using 3D printing technologies, where cell function and viability are preserved within the printed construct. Generally, 3D bioprinting utilizes the layer-by-layer method to deposit materials known as Bioinks to create tissue-like structures that are later used in medical and tissue engineering fields. There are currently at least three types of 3D bioprinting technologies: laser printing technology, micro extrusion or extrusion technology and inkjet technology. In addition to these technologies, acoustic printer and hybrid printing technologies are emerging.

"Bioinks" are materials that mimic an extracellular matrix environment to support the adhesion, proliferation, and differentiation of living cells. Depending on the bioprinting technology that is used, the bioink may or may not comprise cells. For instance, micro-droplets of bioink containing both a gel and cells may be projected on a support. As an alternative, the bioink may not contain the cells and then the bioprinter may work with two printheads, one depositing the gel and the other the cells. The cells are pushed through a micro syringe and deposited using a needle. The layers are alternately deposited, a hydrogel layer (water mixture) followed by a layer of cells. The hydrogel is used to structure the assembly of cell layers, similar to scaffolding.

Advantageously, the bioink may be a gel, preferably selected from hydrogels and supramolecular gels.

"Thixotropy" is a time-dependent shear thinning property. A thixotropic fluid is a fluid which takes a finite time to attain equilibrium viscosity when introduced to a steep change in shear rate. Thixotropic fluids can take various periods of time to return to a gel state.

Advantageously, the gels according to the invention show a thixotropic profile in rheology. The gel may return to its initial state after a high shear stress in about less than 5 minutes. For example, in the context of the invention, bioinks take less than 5 minutes to return to their initial state after a high shear stress, preferably between 5 seconds and 2 minutes.

The "storage modulus" (G'), also called "elastic modulus" or "Young's modulus", corresponds to the amount of energy stored and released in each oscillation. The "loss modulus" (G") or viscous modulus describes the dissipated energy as heat which is lost.

Advantageously, G' values exceed G" values over the entire frequency range, showing the solid character of these materials. In the case of the present invention, gels may have G' values comprised between 150 Pa and 150 kPa and G" values comprised between 100 Pa and 20 kPa. For example, before extrusion, G' values may be comprised between 150 and 3 kPa and G" values may be comprised between 100 Pa and 1.2 kPa. Gels may also have at room temperature (about 25°C) and after extrusion, G' values comprised between 300 Pa and 150 kPa and G" values comprised between 150 Pa and 15 kPa. Gels may also have at physiological temperature (about 37°C) and after extrusion, G' values comprised between 300 Pa and 20 kPa and G" values comprised between 150 Pa and 7 kPa.

"Tsol-gel" is the temperature of transition of a sol to a gel at the gel point.

Advantageously, the gels according to the invention have a Tsol-gel comprised between 25 and 45°C, preferably between 35 and 42°C.

Advantageously, the gel may be a hydrogel. It is meant by "hydrogel", a gel in which the swelling agent is water.

The hydrogel may comprise between 0.05 and 15 % w/w of an aqueous solution, advantageously between 0.5 and 10% w/w.

The aqueous solution may comprise a salt chosen in the group comprising lithium, sodium, potassium, rubidium, cesium, or ammonium chloride, preferably sodium chloride. The concentration of lithium, sodium, potassium, rubidium, cesium, or ammonium chloride in the aqueous solution may be comprised between 0.5 to 1.5 wt.%, preferably is 0.9 wt. %.

Advantageously, the aqueous solution may comprise a salt chosen in the group comprising lithium, sodium, potassium, rubidium, cesium, or ammonium chloride, preferably sodium chloride. The concentration of lithium, sodium, potassium, rubidium or cesium in the aqueous solution may be comprised between 0.5 to 1.5 wt.%, preferably is 0.9 wt. %.

Advantageously, the aqueous solution may be a 0.9% aqueous sodium chloride solution.

Advantageously, the concentration of compound of formula I, II, III, IIIa, IIIb or IIIc in the gel may be comprised between 0.1 and 10 % w/w, preferably between 2 and 5 % w/w and more preferably 3 % w/w.

Advantageously, the gel may further comprise a cell culture medium. The cell culture medium may be selected from the Dulbecco's Modified Eagle Medium (DMEM), the enriched Dulbecco's Modified Eagle Medium (eDMEM), RPMI (Roswell Park Memorial Institute), MEM (Minimum Essential Media), IMDM (Iscove's Modified Dulbecco's Medium), opti-MEM medium (Reduced Serum Media is a modification of Eagle's Minimum Essential Media, buffered with HEPES and sodium bicarbonate, and supplemented with hypoxanthine, thymidine, sodium pyruvate, L-glutamine, trace elements, and growth factors) and DMEM/F-12 (Dulbecco's Modified Eagle Medium: Nutrient Mixture F-12).

Advantageously, the mass ratio aqueous solution / cell culture medium in the gel may be comprised between 10:90 and 50:50, preferably between 20:80 and 40:60, more preferably the ratio is 30:70.

Advantageously, the gel may further comprise cells. The cells may be selected from apical papilla stem cells, human bone marrow cells, human skin fibroblast, human gingival fibroblast, human umbilical vein cells, endothelial cells, hematopoietic stem cells (HSC), human induced pluripotent stem cells (HiPSC) and mesenchymal stem cells (MHSC).

Advantageously, the gel may comprise a concentration of cells between 0.5 million to 5 millions of cells/mL of gel, preferably between 1 and to 5 millions of cells/mL of gel and more preferably between 1 and 2 millionscells/mL of gel.

Advantageously, the bioink formulation according to the invention may be suitable for bioprinting. Preferably the bioprinting method is selected from the group comprising inkjet bioprinting, laser bioprinting and extrusion based bioprinting.

The invention further relates to a bioprinting method comprising the use of a bioink formulation comprising a nucleolipid-based compound of formula I, II, III, IIIa, IIIb or IIIc.

The invention also relates to a bioprinting method comprising a step of (preferably layer by layer) deposition of a bioink formulation comprising a nucleolipid-based compound of formula I, II, III, IIIa, IIIb or IIIc.

The invention may also relate to nucleolipid-based compounds of formula I, II, III, IIIa, IIIb or IIIc for use in regenerative medicine, preferably for use in the bioprinting of vascular niches for cells, wherein the bioprinting is chosen in the group comprising inkjet bioprinting, laser bioprinting and extrusion based bioprinting.

On another aspect, the invention also concerns bioprinted grid or prototype construct comprising nucleolipid-based compounds of formula I, II, III, IIIa, IIIb or IIIc.

The invention is also linked to a bioink gel formulation, preferably a hydrogel or a supramolecular gel, comprising a compound of formula I, II, III, IIIa, IIIb or IIIc, aqueous lithium, sodium, potassium, rubidium, cesium, or ammonium chloride solution, a cell culture medium and optionally cells.

Advantageously, the invention is also linked to a bioink gel formulation, preferably a hydrogel or a supramolecular gel, comprising a compound of formula I, II, III, IIIa, IIIb or IIIc, aqueous lithium, sodium, potassium, rubidium, or cesium solution, a cell culture medium and optionally cells.

Advantageously, the bioink gel formulation, preferably a hydrogel or a supramolecular gel, may comprise:
- between 0.1 and 10 % w/w of a compound of formula I, II, III, IIIa, IIIb or IIIc,
- between 0.1 and 10 %w/w of a 0.5 to 1.5 wt. % aqueous lithium, sodium, potassium, rubidium, cesium, or ammonium chloride solution,
- a cell culture medium, and
- optionally cells,
wherein the ratio aqueous solution / cell culture medium is comprised between 50:50 and 90:10.

Advantageously, the bioink gel formulation, preferably a hydrogel or a supramolecular gel, may comprise:
- between 0.1 and 10 % w/w of a compound of formula I, II, III, IIIa, IIIb or IIIc,
- between 0.1 and 10 %w/w of a 0.5 to 1.5 wt. % aqueous lithium, sodium, potassium, rubidium or cesium solution,
- a cell culture medium, and
- optionally cells,
wherein the ratio aqueous solution / cell culture medium is comprised between 50:50 and 90:10.

### Brief description of the figures

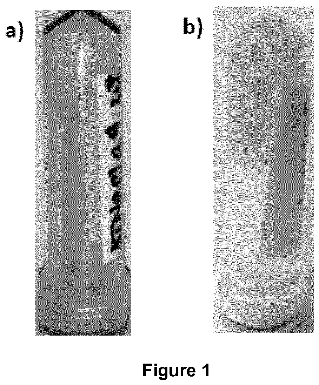

**Figure 1** represents images of hydrogels formed with (a) diC16-3'-dT in 0.9% sodium chloride aqueous solution at 3% (w/v), (b) diC16-3'-dT in 0.9% sodium chloride enriched DMEM (eDMEM) at 3% (w/v).

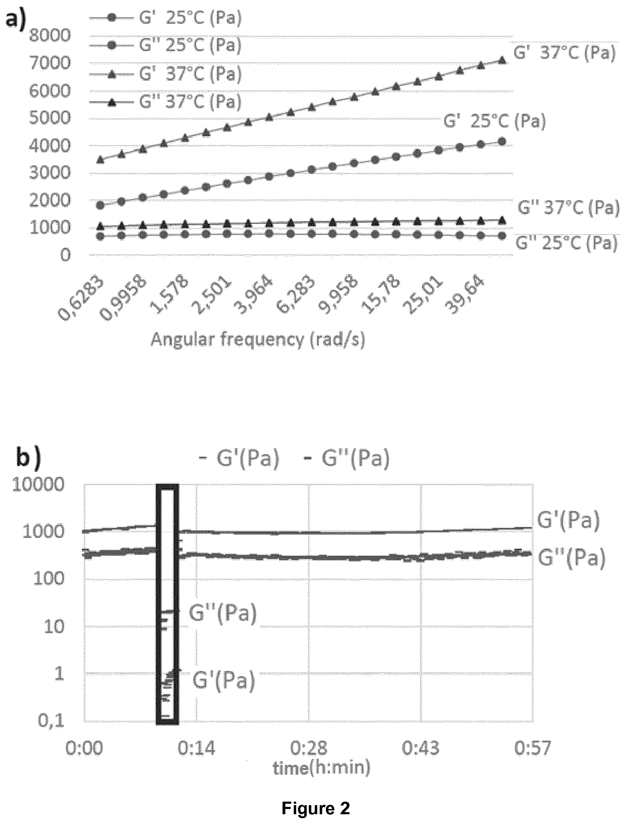

**Figure 2** represents oscillatory rheological experiments of diC16-3'-dT-based gel in eDMEM (enriched DMEM) at 3% (w/v). (a) Frequency sweep results at ambient (25±0.01°C) and physiological (37±0.01°C) temperatures. (b) Step-strain experiment at a fixed angular frequency (6.283 rad/s). The gel was swept from 0.1% to 15% then back to 0.1% strain.
**Figure 3** represents bioprinted grids of (a) diC16-3'-dT-0.9% NaCl aqueous solution hydrogel; 1 layer (b) diC16-3'-dT-0.9% NaCl aqueous **solution** hydrogel; 5 layers (c) diC16-3'-dT in 0.9% sodium chloride enriched DMEM (70/30% v/v) (eDMEM) hydrogel; 1 layer (d) diC16-3'-dT-eDMEM hydrogel; 5 layers.
**Figure 4** represents pictures of bioprinted grid pore of (a) diC16-3'-dT-0.9% NaCl aqueous solution-based gel and (b) diC16-3'-dT in 0.9% sodium chloride enriched DMEM (70/30% v/v) gel (magnification x2.5) (scale bars: 500µm).
**Figure 5** represents confocal microscopy images of Live/Dead® tests at different times (magnification x10) (scale bars: 100µm).
**Figure 6** represents (a) Alamar Blue® representations of D1, D3 and D7 values against blank and (b) a trend curve of Alamar Blue® values at D1, D3 and D7 against D1.
**Figure 7** represents the synthesis scheme of nucleolipids diC16-3'-dG (**1a**) and diC16-3'-dC **(1b).**

### EXAMPLES

### Example 1:

### Materials

The different aqueous solvents were purchased from the following companies: 0.9% sodium chloride aqueous solution (Fresenius Kabi, Bad Homburg vor der Höhe, Germany); Dulbecco's Modified Eagle Medium (DMEM) with low glucose, GlutaMAX® and pyruvate (Gibco®, ThermoFisher Scientific, Walthman, Massachusetts, USA). Milli-Q® water was obtained by filtration through a PURELAB® ultra ELGA (Veolia Water, Antony, France) system with a 0.2µm filter at the water outlet point.

### Nucleolipid-based compounds

Nucleolipids were synthesized according to the procedure described in WO 2009/098404, in particular diC16-3'-dT. 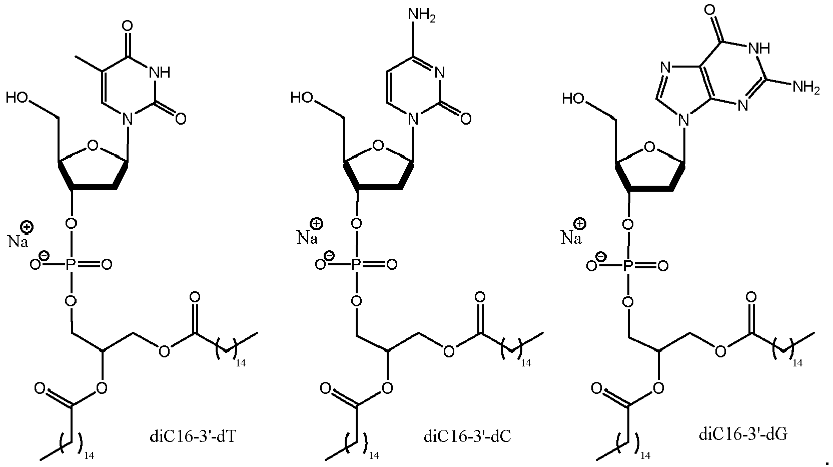 Nucleolipids diC16-3'-dG and diC16-3'-dC were synthesized according to the following procedures.

### Synthesis of triethylammonium (2R,3S,5R)-5-(2-amino-6-oxo-1,6-dihydro-9H-purin-9-yl)-2-(hydroxymethyl)tetrahydrofuran-3-yl((R)-2,3-bis(palmitoyloxy)propyl)phosphate(diC16-3'-dG)(compound 1a)

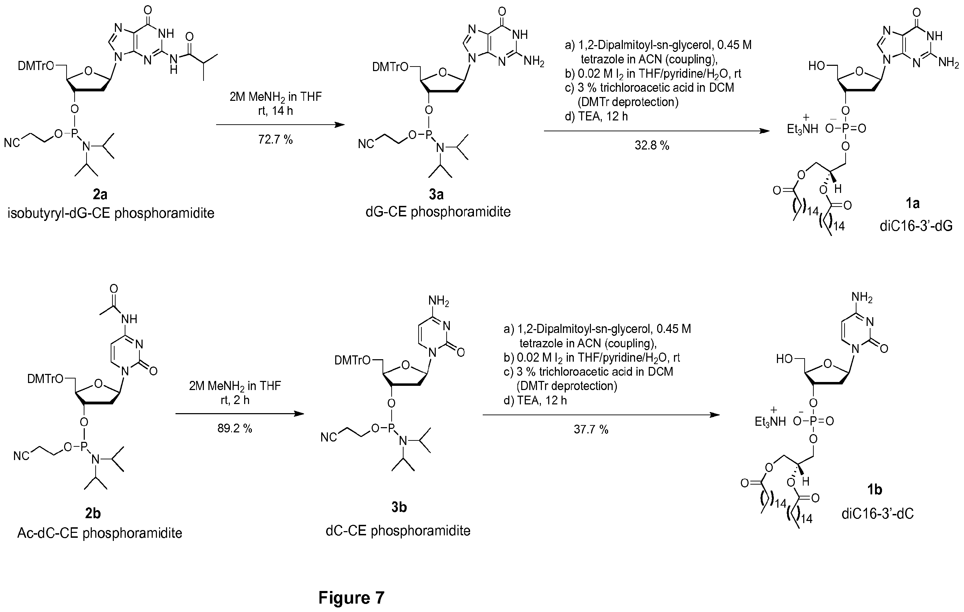

The synthesis scheme is represented on Figure 7.
a) *Step 1 (deprotection of isobutyryl group):* (2*R*,3*S*,5*R*)-2-((bis(4-methoxyphenyl)(phenyl)methoxy)methyl)-5-(2-isobutyramido-6-oxo-1,6-dihydro-9*H*-purin-9-yl)tetrahydrofuran-3-yl (2-cyanoethyl) diisopropyl-phosphoramidite (isobutyryl-dG-CE phosphoramidite) **2a** (2 g, 2.38 mmol, 1 equiv.) was dissolved in MeNH₂ (2 M in THF, 23.81 mL, 47.6 mmol, 20 equiv.) at room temperature under argon. After being stirred for 14 h, the mixture was evaporated under reduced pressure. The completion of the reaction was confirmed by TLC (disappearance of the spot corresponds to starting material and appearance of a new spot at slightly lower retardation factor (*R_{f}*)) The residue was chromatographed on a column of silica gel with 1% MeOH/DCM (v/v) containing 1% TEA and then 3% MeOH/DCM (v/v) containing 1% TEA to give the fractions containing the target (2R,3S,5R)-5-(2-amino-6-oxo-1,6-dihydro-9H-purin-9-yl)-2-((bis(4-methoxyphenyl)(phenyl)methoxy)methyl)-tetrahydrofuran-3-yl (2-cyanoethyl) diisopropylphosphoramidite **3a.**
   Yield: 1.33 g (72.7 %). The product was utilized for next step without further characterization.
b) *Step 2 (coupling with lipid):* (2R,3S,5R)-5-(2-amino-6-oxo-1,6-dihydro-9*H*-purin-9-yl)-2-((bis(4-methoxyphenyl)(phenyl)methoxy)methyl)-tetrahydrofuran-3-yl (2-cyanoethyl) diisopropylphosphoramidite **3a** (product of step 1) (1.2 g, 1.64 mmol, 1 equiv.) and 1,2-dipalmitoyl-*sn-*glycerol (1.21 g, 2.14 mmol, 1.3 equiv,) were dissolved in dry THF (25 mL). A tetrazole solution in acetonitrile (0.45 M, 6.2 mL, 2.8 mmol, 1.7 equiv.) was added, and the reaction mixture was stirred overnight at room temperature followed by oxidation with I₂ solution (0.02 M in THF/Pyridine/H₂O, 140 mL, 2.8 mmol, 1.7 equiv.). After 6 h at room temperature, the solvent was evaporated under high vacuum and the intermediate products were dissolved in ethyl acetate (100 mL) and then washed with saturated Na₂S₂O₃ solution (2 x 25 mL). The fractions were dried over anhydrous sodium sulfate, and the solvent was evaporated. The resulting product was dissolved in DCM (20 mL) and trichloroacetic acid (TCA, 3% in DCM) solution (15 mL) under argon, after 4 h at room temperature, 5 mL of methanol and 25 mL of sodium hydrogen carbonate solution were added. The aqueous phase was extracted with DCM (2 x 30 mL), and then the organic fractions were collected, dried over anhydrous sodium sulfate, and the solvent evaporated. The intermediate product was dissolved 33% trimethylamine (TEA) in DCM (50 mL DCM + 25 mL TEA) and stirred overnight at room temperature (to ensure the complete deprotection of cyanoethyl chain). The solvent was evaporated under high vacuum.

The product **1a** was isolated after purification on silica gel (DCM:methanol:TEA from 98:1:1 to 90:9:1). Yield: 852 mg (32.8 %). *R_{f}* (product **1a**) = 0.3 (DCM:methanol:TEA 90:9:1).

**¹H NMR** (300 MHz, CDCl₃): *δ* in ppm 0.86 (t, 6H, J=6.6 Hz, 2CH₃ of palmitoyl chain), 1.23 (s, 48H, 24CH₂ (palmitoyl chain)), 1.33 (t, 9H, J=7.2 Hz, 3CH₃ (triethylammonium)), 1.57 (bs, 4H, 2*CH₂*-CH₂-CO), 2.13-2.40 (m, 4H, 2*CH₂*-CO), 2.54 (bs, 1 H, H2'(sugar)), 2.87 (bs, 1 H, H2"(sugar)), 3.11 (q, J=7.1, 3CH₂ (triethylammonium)), 3.64-4.49 (m, 8H, 2CH₂ (glycerol), H3' (sugar), H4'(sugar), 2H5'(sugar)), 5.03-5.36 (m, 2H, -CH-glycerol, -OH), 6.16 (t, 1H, J=6.8 Hz, H1'), 6.52 (bs, 2H, -NH₂), 7.67 (s, 1 H, H-8(base)), 12.42 (s, 1 H, -NH(base)).

**¹³C NMR** (75 MHz, CDCl₃): *δ* in ppm 8.7 (CH₃, triethylammonium), 14.2 (CH₃ chain), 22.8 (*CH₂*-CH₃ chain), 25.0 (*CH₂*-CH₂-C=O), 29.2-29.8 (CH₂ chain), 32.0 (*CH₂*-CH₂-CH₃ chain), 34.2 (*CH₂*-C=O chain), 34.4 (*CH₂*-C=O chain), 45.9 (CH₂ triethylammonium), 62.7(*CH₂*-O-C=O glycerol), 63.2 (*CH₂*-O-P=O glycerol), 63.7 (CH₂, C5'-sugar), 70.35 (CH, C3'-sugar), 70.44 (CH glycerol), 86.7 (C, C4'-sugar), 87.7 (CH, C1'-sugar), 118.6 (C, C5-base), 137.0 (CH, C8-base), 149.9 (C, C4-base), 154.0 (C, C2-base), 158.2 (C=O, C6-base), 173.2 (C=O chain), 173.5 (C=O chain).

**³¹P NMR** (121 MHz, CDCl₃): *δ* in ppm 1.86. High-resolution ESI MS
[M-H]⁻, theoretical m/z=896.55192, observed m/z=896.5525 (1 ppm).

### Synthesis of triethylammonium (2R,3S,5R)-5-(4-amino-2-oxopyrimidin-1(2H)-yl)-2-(hydroxymethyl)tetrahydrofuran-3-yl ((R)-2,3-bis(tetradecanoyloxy)propyl) phosphate(diC16-3'-dC) (compound 1b)

The synthesis scheme is represented on Figure 7.
a) *Step 1 (deprotection of isobutyryl group):* (2*R*,3*S*,5*R*)-5-(4-acetamido-2-oxopyrimidin-1(2*H*)-yl)-2-((bis(4-methoxyphenyl)(phenyl)-methoxy)methyl)tetrahydrofuran-3-yl (2-cyanoethyl) diisopropyl-phosphoramidite (Ac-dC-CE phosphoramidite) **2b** (2 g, 2.6 mmol, 1 equiv.) was dissolved in MeNH₂ (2 M in THF, 25.9 mL, 51.8 mmol, 20 equiv.) at room temperature under argon. After being stirred for 2 h, the mixture was evaporated under reduced pressure. The completion of the reaction was confirmed by TLC (disappearance of the spot corresponds to starting material and appearance of a new spot at slightly lower retardation factor (*R_{f}*)). The residue was chromatographed on a column of silica gel with 1% MeOH/DCM (v/v) containing 1% TEA and then 3% MeOH/DCM (v/v) containing 1% TEA to give the fractions containing the target (2R,3S,5R)-5-(4-amino-2-oxopyrimidin-1 (2H)-yl)-2-((bis(4-methoxyphenyl)(phenyl)methoxy)methyl)tetrahydrofuran-3-yl (2-cyanoethyl) diisopropylphosphoramidite **3b**.
   Yield: 1.68 g (89.2 %). The product was utilized for next step without further characterization.
b) *Step 2 (coupling with lipid):* (2R,3S,5R)-5-(4-amino-2-oxopyrimidin-1(2*H*)-yl)-2-((bis(4-methoxyphenyl)(phenyl)methoxy)methyl)-tetrahydrofuran-3-yl (2-cyanoethyl) diisopropylphosphoramidite **3b** (product of step 1) (1.4 g, 1.81 mmol, 1 equiv.) and 1,2-dipalmitoyl-*sn-*glycerol (1.34 g, 2.36 mmol, 1.3 equiv,) were dissolved in dry THF (25 mL). A tetrazole solution in acetonitrile (0.45 M, 6.9 mL, 3.1 mmol, 1.7 equiv.) was added, and the reaction mixture was stirred overnight at room temperature followed by oxidation with I₂ solution (0.02 M in THF/Pyridine/H₂O, 154 mL, 3.1 mmol, 1.7 equiv.). After 6 h at room temperature, the solvent was evaporated under high vacuum and the intermediate products were dissolved in ethyl acetate (100 mL) and then washed with saturated Na₂S₂O₃ solution (2 x 25 mL). The fractions were dried over anhydrous sodium sulfate, and the solvent was evaporated. The resulting product was dissolved in DCM (20 mL) and trichloroacetic acid (TCA, 3% in DCM) solution (15 mL) under argon, after 4 h at room temperature, 5 mL of methanol and 25 mL of sodium hydrogen carbonate solution were added. The aqueous phase was extracted with DCM (2 x 30 mL), and then the organic fractions were collected, dried over anhydrous sodium sulfate, and the solvent evaporated. The intermediate product was dissolved 33% trimethylamine (TEA) in DCM (50 mL DCM + 25 mL TEA) and stirred overnight at room temperature (to ensure the complete deprotection of cyanoethyl chain). The solvent was evaporated under high vacuum.

The product **1b** was isolated after purification on silica gel (DCM:methanol:TEA from 98:1:1 to 90:9:1).

Yield: 653 mg (37.7 %). *R_{f}* (product **1b**) = 0.3 (DCM:methanol:TEA 90:9:1).

**¹H NMR** (300 MHz, CDCl₃): *δ* in ppm 0.85 (t, 6H, J=6.5 Hz, 2CH₃ of palmitoyl chain), 1.22 (s, 48H, 24CH₂ (palmitoyl chain)), 1.27 (t, 9H, J=7.4 Hz, 3CH₃ (triethylammonium)), 1.55 (bs, 4H, 2*CH₂*-CH₂-CO), 2.00-2.34 (m, 5H, 2*CH₂*-CO, H2'(sugar)), 2.51 (bs, 1 H, H2"(sugar)), 3.02 (q, J=7.2, 3CH₂ (triethylammonium)), 3.25-4.91 (m, 8H, 2CH₂ (glycerol), H3' (sugar), H4'(sugar), 2H5'(sugar)), 5.21 (bs, 1 H, -CH- glycerol), 6.02 (d, 1 H, J=6.5 Hz, H-5(base)), 6.12 (t, 1 H, J=5.3 Hz, H1'), 8.05 (bs, 1 H, H-6 (base)).

**¹³C NMR** (75 MHz, CDCl₃): *δ* in ppm 9.0 (CH₃, triethylammonium), 14.2 (CH₃ chain), 22.8 (*CH₂*-CH₃ chain), 25.0 (*CH₂*-CH₂-C=O), 29.3-29.8 (CH₂ chain), 32.0 (*CH₂*-CH₂-CH₃ chain), 34.2 (*CH₂*-C=O chain), 34.4 (*CH₂*-C=O chain), 45.9 (CH₂ triethylammonium), 61.4 (CH₂, C5'-sugar), 62.8 (*CH₂*-O-C=O glycerol), 63.6 (*CH₂*-O-P=O glycerol), 70.4 (CH glycerol), 74.8 (CH, C3'-sugar), 86.4 (C, C4'-sugar), 95.4 (CH, C5-base), 95.5 (CH, C1'-sugar), 142.1 (CH, C6-base), 155.1 (C=O, C2-base), 162.5 (C, C4-base), 173.2 (C=O chain), 173.6 (C=O chain).

**³¹P NMR** (121 MHz, CDCl₃): *δ* in ppm 1.90. High-resolution ESI MS [M-H]⁻, theoretical m/z=856.54577, observed m/z=856.5459 (0 ppm).

### Hydrogel bioink formulations

Hydrogels formulations of diC16-3'-dT, diC16-3'-dC, diC16-3'-dG were prepared according to previous work **[17-19].**

In order to evaluate gel fixation (i.e. gelation), samples were turned upside-down; remaining still under its own weight indicated a complete gelation.

### Preparation of diC16-3'-dT, diC16-3'-dC and diC16-3'-dG hydrogels

diC16-3'-dT, diC16-3'-dC or diC16-3'-dG was mixed with 0.9% sodium chloride aqueous solution, DMEM and DMEM/0.9% sodium chloride aqueous solution (70/30% v/v) called enriched DMEM (eDMEM) to make 3% (w/v) gels. eDMEM osmolarity was controlled with a Type 15 osmometer (Fisher Scientific SAS, Illkirch Graffenstaden, France). Samples were heated at 55°C and stirred at 1000 rpm for 30 minutes. Samples were then stored at room temperature.

### Example 2:

### Characterization

### Determination of hydrogels mechanical properties by rheology.

Rheological experiments were performed using a Malvern Kinexus Pro+ rheometer (Malvern Instrument SA, Worcestershire, United Kingdom) with a steel cone-plate geometry (diameter: 20mm; angle: 1°).

A solvent trap was used to prevent solvent evaporation and to control temperature. The low plate was equipped with a Peltier temperature control system. Samples of hydrogels of example 1 were studied at ambient (25 ± 0.01°C) and physiological (37 ± 0.01°C) temperatures. Materials were placed on the low geometry under their gel states using a plastic spatula and allowed to rest 30 minutes before any experiments. All measurements were carried out within the linear viscoelastic regime (LVR). It was determined by amplitude strain sweeps from 0.01 to 100% at an angular frequency of 1 Hz (6.283 rad.s-1). Storage modulus (G') and loss modulus (G") were studied using a frequency sweep test ranging from 0.1Hz to 10Hz.

To determine the transition temperature (Tsol-gel), a single frequency stress controlled temperature ramp was performed. This sol-gel transition temperature was determined at 1Hz frequency and 1Pa strain. The ramp was included between 25 and 90°C (heating rate of 2°C/min).

Thixotropy was assessed by subjecting gels to low/high strain cycles. Gel was first subjected to a low strain (0.1%, into its LVR) for 10 minutes. Then, the sample was suddenly subjected to a higher strain (15%, beyond its LVR) for 2 minutes. Last, the strain came back to the first step value (0.1%, into its LVR) until full recovery of its initial properties.

The values of G' and G" are compiled in the tables below.

**Table 1: Shear moduli (G' and G") of 3 %w/v diC16-3'-dT-based gels formed in 0.9% sodium chloride aqueous solution or in eDMEM (enriched DMEM) at ambient temperature (25±0.01°C) before and after syringe pump extrusion with a 22 gauge needle, expressed as mean ± standard deviation of the mean.**

| diC16-3'-dT | Before extrusion | | After extrusion | |
|---|---|---|---|---|
| 25°C | 0.9% sodium chloride | eDMEM | 0.9% sodium chloride | eDMEM |
| G' (Pa) | 1945 ± 549 | 362 ± 176 | 576 ± 171 | 2993 ± 1591 |
| G" (Pa) | 732 ± 200 | 269 ± 144 | 289 ± 105 | 784 ± 337 |

**Table 2: Shear moduli (G' and G") of 3 %w/v diC16-3'-dT-based gels formed in eDMEM (0.9% sodium chloride aqueous solution enriched DMEM) at physiological temperature (37±0.01°C) before and after syringe pump extrusion with a 22 gauge needle, expressed as mean ± standard deviation of the mean.**

| diC16-3'-dT-eDMEM 37°C | Before extrusion | After extrusion |
|---|---|---|
| G' (Pa) | 675 ± 417 | 5222 ± 1144 |
| G" (Pa) | 340 ± 230 | 1189 ± 176 |

**Table 3 : Shear moduli (G' and G") of 3 %w/v diC16-3'-dT-based gels formed in eDMEM (0.9% sodium chloride aqueous solution enriched DMEM) at ambient (25±0.01°C) and physiological (37±0.01°C) temperatures after syringe pump extrusion with a 22 gauge needle and 21 days stability in a 37°C dried incubator, expressed as mean ± standard deviation of the mean.**

| diC16-3'-dT- eDMEM | 25°C | 37°C |
|---|---|---|
| G'(Pa) | 112133 ± 1305 | 39220 ± 1160 |
| G" (Pa) | 14430 ± 324 | 5181 ± 453 |

### Example 3:

### Preliminary structural stability

A preliminary structural stability of diC16-3'-dT construction was carried out with a UFE400 heating oven (Memmert GMBH CO KG, Schwabach, Deutschland) for 21 days. Samples were checked up at Day D0, D1, D2, D3, D7, D14 and D21. Two series of the same batch were incubated in glass vials at 37°C to mimic cell culture conditions within 3 days. One series consisted in 6 samples of syringe pump extruded gel. 3 samples per series were put in a 2ml bath of DMEM while the other three were stored without bath.

A semi quantitative assessment was carried out to evaluate degradation and release of diC16-3'-dT into DMEM bath, 6µl of bulk solution were removed for each samples covered up by DMEM and analysed in triplicate with a micro-volumes spectrophotometer mySPEC (VWR International, Fontenay-sous-Bois, France).

Structural stability of hydrogel construct is required for cell culture as it was aimed to have 21 days stability of the scaffold with cell-laden hydrogel after bioprinting. Macroscopic stability was assessed by a syringe pump extrusion-based construction of diC16-3'-dT-eDMEM hydrogel covered by DMEM.

After 21 days, visual observations showed dehydrated constructs for samples without a bath of DMEM. Gels placed in DMEM visually showed a harder structure after 21 days. These observations were confirmed by rheological experiments carried out at ambient and physiological temperatures. G' modulus increased drastically compared to day 0 (table 3). These moduli were almost height times higher for eDMEM solvent and 35 times higher for 0.9% sodium chloride aqueous solution. G' values were statistically different (p<0.0001) for both conditions i.e. after extrusion and after stability study reflecting a concentration of the gel.

### In vitro cell assays - Gingival fibroblasts culture

The human gingival fibroblast (FGh) was chosen as a stromal cell which viability could resist to the extrusion stress. Human gingival fibroblasts cultures were obtained from gingival explants of healthy non-smoker patients with no history of periodontitis. All patients gave their informed consent according to the Helsinki Declaration of 1975, and denied having recently taken drugs that could affect connective tissue metabolism. Briefly, gingival explants were collected from surgical wastes under local anaesthesia and conserved in DMEM completed with 20% foetal calf serum (FCS), 100 Ul/ml penicillin, 100 µg/ml streptomycin, and 2.5 µg/ml amphotericin B (all from Invitrogen).

Explants were put in culture flasks and the medium was changed every 2 days. After the first passage, FGhs were grown in DMEM completed with 10% FCS and used from passages 3 to 5.

### In vitro cell viability

Cell viability was controlled at D1, D3, D7, D14 and D21 with a Live/Dead® test (Thermofisher Scientific, Waltham, MA, USA). In living cells, non-fluorescent substrate acetoxymethyl calcein (AMC) is cleaved into green fluorescent calcein by an intracellular esterase. Dead cells which membranes are compromised allow intercalating agent ethidium homodimer-1 (EDT-1) to pass inside cell cores and so amplifying red fluorescence. The Live/Dead® solution was prepared as follow: an aliquot of AMC and an aliquot of EDT-1 were mixed with 2.5ml of DMEM and heated in a boiling water bath for 15 minutes sheltered from light.

Two series of 5 samples of 100µL of diC16-3'-dT hydrogel in eDMEM were seeded with FGh (2.106 cells/ml) and deposited in a 12-well plate. At each time, sample was sacrificed and incubated (15 minutes, 37°C, 5% CO2) then washed with DPBS 1X (Gibco®). A confocal microscope (TCS SPE, CTR 6500, Leica, Wetzlar, Germany) was used for observations and wavelengths were fixed at 488 nm for living cells and 561 nm for dead cells.

### In vitro cellular activity

An Alamar Blue® (ThermoFischer Scientific, Waltham, MA, USA) test was performed to assess cellular activity at D1, D3, D7, D14 and D21. Alamar Blue® reagent is a cellular health indicator using the reducing power of living cells to quantitatively measure the spread of multi lineage cells. Briefly, when cells are alive, they maintain a reduced environment into cytosol. Resazurine, active ingredient of Alamar Blue® reagent, is a non-toxic non-fluorescent cell permeability blue dye. Inside cells, resazurine is reduced into resofurine which is a red fluorescent component. Living cells convert continuously resazurine into resofurine, enhancing the total fluorescence.

Two series of 3 samples of 100µl of diC16-3'-dT hydrogels in eDMEM seeded with FGh (2.106 cells/ml) were placed on a 6-well plate and reused for the different times. For each time, hydrogel matrices were washed two times in order to eliminate Alamar Blue® traces. Results were obtained at 590 nm by a spectrophotometer 2030 Multilabel Reader VICTOR X3 (PerkinElmer, Waltham, MA, USA) linked to WorkOut® software.

diC16-3'-dT hydrogel formed in 0.9% sodium chloride aqueous solution already proved minimal inflammatory and fibrosis reactions (19). Cells choice was first headed toward FGh as they were resistant to multiple external aggressions, easy to handle and less expensive than HSCs. Previously studies showed that FGh did not suffer from extrusion, making them a solid layout for preliminary cells assays before testing HSCs.

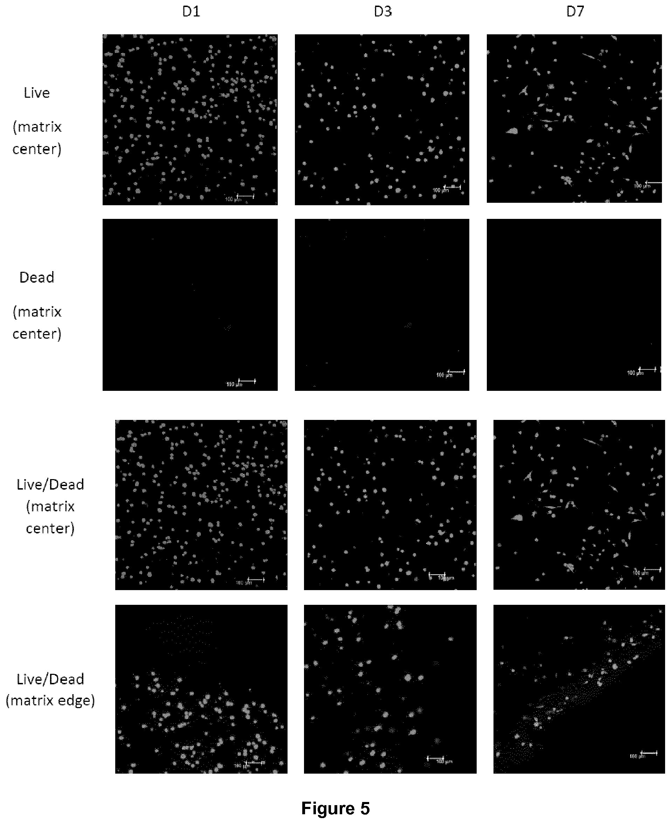

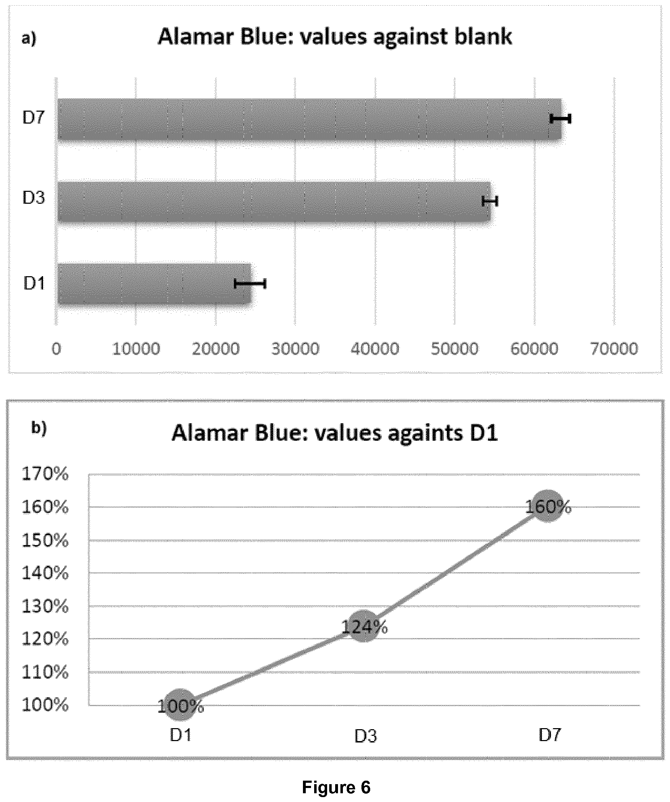

Confocal microscopy observations with Live/Dead stain showed morphological changes. At Day 1 cells were round, consequence of a possible stress but dead cells were practically non-existent. Some FGhs began to show extents at Day 3 and regained their spindle-shape at Day 7 (fig. 5). Moreover, cell-cell interactions were visible at Day 7 at the matrix edge. Alamar Blue® assessment, triplicated for each time, showed a progressive increase of cellular activity (fig.6a). This activity improved at each time in comparison to Day 1 (Day 3: 120% of Day 1 activity and Day 7: 160%) (fig.6b).

Those results proved cell culture compatibility of diC16-3'-dT-eDMEM hydrogel and assumed sufficient porosity of the gel for oxygen and nutrients to reach cells.

Printing of diC16-3'-dT-eDMEM hydrogel was successful and FGh showed spreading and interactions inside the gel.

### Statistical analysis

To handle reproducibility issues, experiments were performed in triplicate or more. All data were expressed as mean ± standard deviation of the mean (SD). XLstat® (Addinsoft, Paris, France) software was used for all statistical analyses. Distribution normality was assessed with a Shapiro-Wilk test. Student's t-test (2-tailed) with a Fisher variance testing was performed to determine differences between groups. Significance was set at p < 0.05 or less.

### Example 4: 3D-extrusion-based bioprinting

### Printer compatibility testing

Bioink was prior tested on bench using a simple extrusion test with syringe and small gauge needles tip (18 and 22 gauges). Briefly, bioinks were prepared as described above but were loaded in syringe under their liquid states before cooling down or sonication steps. After complete gelation, they were prepared for a syringe pump (Razel Scinetific Instrument, Fairfax, USA) extrusion at a flow rate near to the one of bioprinter (14.5 ml/h). Next, bioinks prepared using the same protocol were directly tested on bioprinter.

### Extrusion-based bioprinter (EBB)

Multiprint bioprinter was an extrusion-based 3D printer designed by the Fablab Coh@bit of Technological University Institute of Bordeaux University (IUT). The system was mechanical as material was pushed out the syringe using a piston handled by an endless screw adapted for glass-metal syringes (glass body; metal piston) of three different volumes: 1, 3, 5ml. Straight and non-bevelled luer-lock needles of 18 and 22 gauges diameters were used. For computer-aided design (CAD), multiprint was linked to BioPrinter® software and printing templates were generated by SketchUp software® (Trimble Inc., Sunnyvale, CA, USA). Two CAD designs were used: cube and grid with 2x2 square millimeters pores. Each layer of the construct was composed of approximately 100µL of hydrogel for both patterns.

### 3D extrusion-based bioprinting

Bioink characteristics should meet mechanicals requirements for the EBB bioprinting. Before using the Multiprint 3D bioprinter, hydrogels printability was evaluated by manual syringe pump extrusion.

diC16-3'-dT based bioinks were tested in ether Milli-Q® water, 0.9% sodium chloride aqueous solution and DMEM/eDMEM. diC16-3'-dT regained a gel solid-like shape after extrusion.

Interestingly, after extrusion at room temperature with a syringe pump at a controlled rate, diC16-3'-dT/eDMEM hydrogel (3% w/v) at ambient temperature showed an increase of its G' modulus up to ten times higher. Comparable behaviour was found at physiological temperature before and after extrusion with a syringe pump (p=0.003) (table 2). Extruded hydrogel at physiological temperature showed a comparable G' to perivascular environment in bone marrow niche (about 3 kPa) allowing spreading of HSCs.

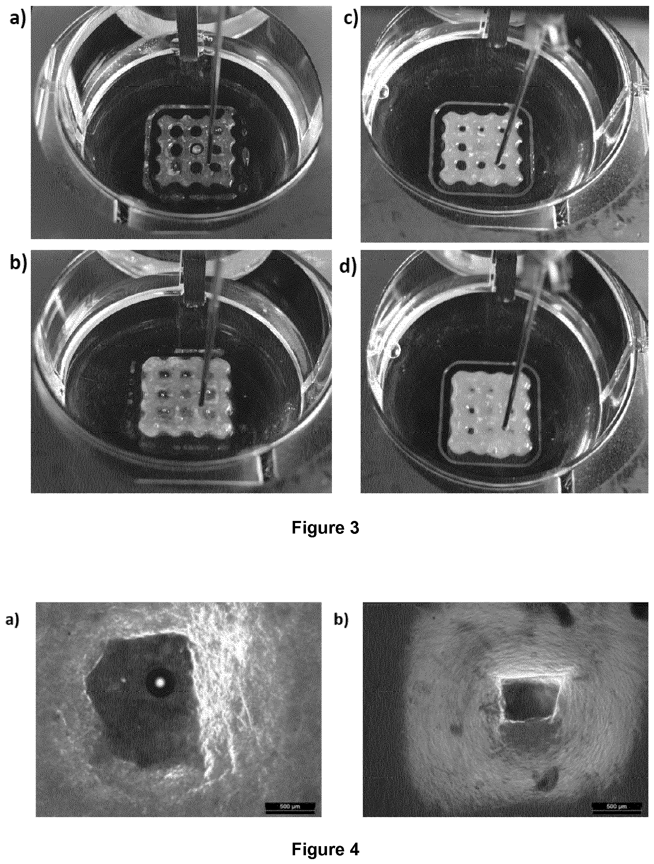

diC16-3'-dT-based hydrogels were then printed using the Multiprint printer into a CAD-grid matrix (fig.3). A pause of 2 minutes was respected between two layers in order to have enough time for the hydrogel reconstruction and prevent grid collapse.

In order to counterbalance the higher viscosity of diC16-3'-dT formed in eDMEM compared to the 0.9% sodium chloride aqueous solution, bioprinting parameters were changed to an increased extrusion coefficient.

Grid is a commonly used matrix in EBB as it showed a perfect compatibility with cells proliferation **[3,21]**. Cells growth was enhanced by pores created into the structure allowing a greater exchange surface between cells and culture medium. Hydrogels constructs showed pores of 1 mm square in 0.9% sodium chloride aqueous solution and smaller ones (500 µm) in eDMEM (fig.4).

Printing of diC16-3'-dT-eDMEM hydrogel was successful and FGh showed spreading and interactions inside the gel.

Applicants successfully bioprinted a bioinspired supramolecular nucleolipid-based hydrogel in culture medium for the first time. Compounds of the invention are suitable molecules for use in supramolecular hydrogel bioink formulations.

The physico-chemical formulations properties allowed forming a stable and cell compatible gel in a culture medium. Rheological properties highlighted high valuable capacities for EBB allowing cells culture and thixotropy providing a biomaterial free of external crosslinkers.

These excellent results combined with in vitro compatibility and biodegradability show that use of nucleolipid based bioink according to the invention is a real improvement in the field of bioinks and bioprinting.

### List of references

[1] Murphy S V, Atala A. 3D Bioprinting of Tissues and Organs. Nat Biotechnol. 2014;32(8):773-85.
[2] Pati F, Gantelius J, Svahn HA. 3D Bioprinting of Tissue/Organ Models. Angew Chemie - Int Ed. 2016;55(15):4650-65.
[3] Ozbolat IT, Hospodiuk M. Current advances and future perspectives in extrusion-based bioprinting. Biomaterials. 2016;76:321-43.
[4] Starly B, Shirwaiker R. Chapter 3 - 3D Bioprinting Techniques [Internet]. 3D Bioprinting and Nanotechnology in Tissue Engineering and Regenerative Medicine. Elsevier Inc.; 2015. 57-77 p.
[5] Yeong W-Y, Chua C-K, Leong K-F, Chandrasekaran M. Rapid prototyping in tissue engineering: challenges and potential. Trends Biotechnol [Internet]. 2004;22(12):643-52.
[6] Hospodiuk M, Dey M, Sosnoski D, Ozbolat IT. The bioink: A comprehensive review on bioprintable materials. Biotechnol Adv [Internet]. 2017;35(2):217-39.
[7] Tibbitt MW, Anseth KS. Hydrogels as extracellular matrix mimics for 3D cell culture. Biotechnol Bioeng. 2009;103(4):655-63.
[8] Skardal A, Atala A. Biomaterials for Integration with 3-D Bioprinting. Ann Biomed Eng. 2015;43(3):730-46.
[9] Bidarra SJ, Barrias CC, Granja PL. Injectable alginate hydrogels for cell delivery in tissue engineering. Acta Biomater. 2014;10(4):1646-62
[10] Antoine EE, Vlachos PP, Rylander MN. Review of Collagen I Hydrogels for Bioengineered Tissue Microenvironments: Characterization of Mechanics, Structure, and Transport. Tissue Eng Part B Rev [Internet]. 2014;20(6):683-96.
[11] Kiyozumi T, Yasuhiro K, Ishihara M, Saitoh D, Shimizu J, Yura H, et al. Medium (DMEM/F12)-Containing Chitosan Hydrogel as Adhesive and Dressing in Autologous Skin Grafts and Accelerator in the Healing Process. J Biomed Mater Res B Appl Biomater. 2005;83(2):340-4.
[12] Steed JW. Supramolecular gel chemistry: developments over the last decade. Chem Commun [Internet]. 2011;47(5):1379-83.
[13] Du X, Zhou J, Shi J, Xu B. Supramolecular Hydrogelators and Hydrogels: From Soft Matter to Molecular Biomaterials. Chem Rev. 2015;115(24):13165-307.
[14] Estroff L a, Hamilton AD. Water Gelation by Small Organic Molecules Water Gelation by Small Organic Molecules. 2004;104(February):1201-18.
[15] Hennink WE, van Nostrum CF. Novel crosslinking methods to design hydrogels. Adv Drug Deliv Rev. 2012;64(SUPPL.):223-36.
[16] Latxague L, Patwa A, Amigues E, Barthélémy P. Glycosyl-nucleolipids as new bioinspired amphiphiles. Molecules. 2013;18(10):12241-63.
[17] Ramin MA, Latxague L, Sindhu KR, Chassande O, Barthélémy P. Low molecular weight hydrogels derived from urea based-bolaamphiphiles as new injectable biomaterials. Biomaterials [Internet]. 2017;1-19.
[18] Latxague L, Ramin MA, Appavoo A, Berto P, Maisani M, Ehret C, et al. Control of Stem-Cell Behavior by Fine Tuning the Supramolecular Assemblies of Low-Molecular-Weight Gelators. Angew Chemie - Int Ed. 2015;54(15):4517-21.
[19] Ramin MA, Sindhu KR, Appavoo A, Oumzil K, Grinstaff MW, Chassande O, et al. Cation Tuning of Supramolecular Gel Properties: A New Paradigm for Sustained Drug Delivery. Adv Mater. 2017;29(13):2-7
[20] WO 2009/098404.
[21] Duan B, Hockaday LA, Kang KH, Butcher JT. 3D Bioprinting of heterogeneous aortic valve conduits with alginate/gelatin hydrogels. J Biomed Mater Res - Part A. 2013;101 A(5):1255-64.

## Claims

1. Use of a nucleolipid-based compound of formula I: 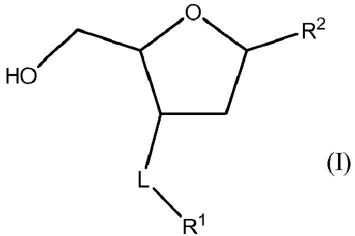 wherein,
- L represents an oxycarbonyl -O-C(O)- group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₂-C₃₀ hydrocarbon chain,
- R¹ represents
o a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether, or
o a diacylglycerol (or diglyceride) in which each acyl chain is a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether,
- R² represents a purine or pyrimidine base selected from uracil, adenine, guanine, cytosine, thymine, hypoxanthine, or their derivatives, optionally substituted, or a non-natural mono- or bicyclic heterocyclic base each ring of which comprising form 4 to 7 members, preferably guanine, cytosine or thymine and more preferably thymine, and
- the compound is optionally in the form of a salt, preferably a metal cation salt and more preferably the metal cation may be selected from Li⁺, Na⁺, K⁺, Rb⁺, Cs⁺,
in a bioink formulation.

2. Use according to claim 1, wherein the nucleolipid-based compound is of formula II: 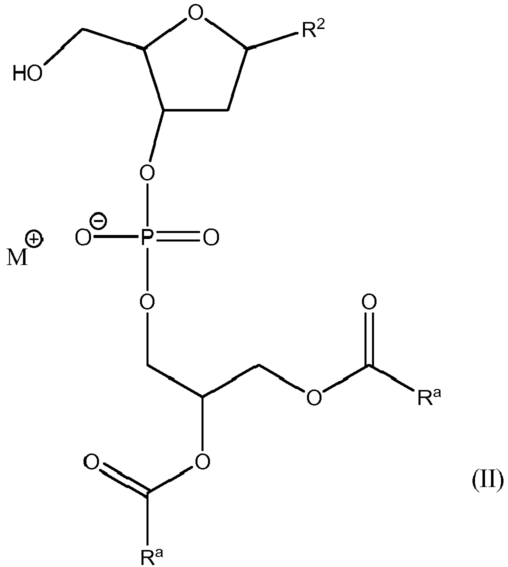 wherein,
- each R^{a}, identical or different, is a linear or branched C₁ to C₂₉ hydrocarbon chain, preferably C₅ to C₂₄ and more preferably C₇ to C₂₄, saturated or unsaturated,
- R² represents a purine or pyrimidine base selected from uracil, adenine, guanine, cytosine, thymine, hypoxanthine, or their derivatives, optionally substituted, preferably guanine, cytosine or thymine and more preferably thymine, and
- M⁺ represents a cation, preferably a metal cation, and more preferably selected from Li⁺, Na⁺, K⁺, Rb⁺, Cs⁺.

3. Use according to claim 2, wherein each R^{a}, identical or different, is a C₁₃ to C₁₉ linear hydrocarbon chain, preferably a C₁₅ linear hydrocarbon chain.

4. Use according to any of the preceding claims, wherein the nucleolipid-based compound is of formula III: 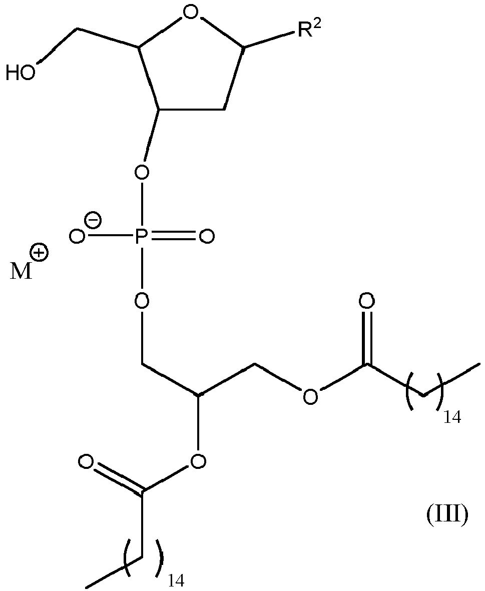 wherein R² represents a purine or pyrimidine base selected from guanine, cytosine and thymine, preferably R² represents a pyrimidine base and more preferably R² represents thymine and M⁺ is as defined in claim 2.

5. Use according to any of the preceding claims, wherein nucleolipid-based compound is selected from compounds of formula IIIa, IIIb and IIIc, preferably of formula IIIa: 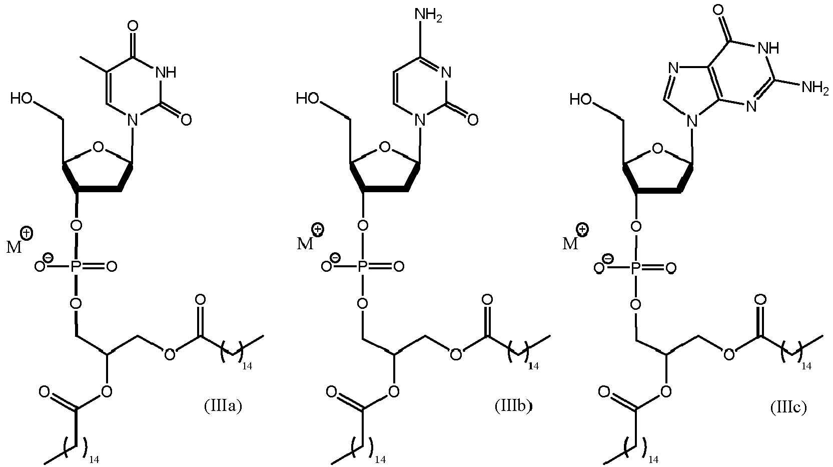 M⁺ being as defined in claim 2.

6. Use according to any of the preceding claims, wherein the bioink is a gel, preferably selected from hydrogels and supramolecular gels.

7. Use according to the preceding claim, wherein the gel is a hydrogel and further comprises a 0.5 to 1.5 wt. % aqueous lithium, sodium, potassium, rubidium or cesium solution, preferably a 0.9% aqueous sodium chloride solution.

8. Use according to claim 6 or 7, wherein the concentration of compound of formula I, II, III, IIIa, IIIb or IIIc in the gel is comprised between 0.1 and 10 % w/v, preferably 3 % w/v.

9. Use according to any of claims 6 to 8, wherein the gel further comprises a cell culture medium, preferably selected from the Dulbecco's Modified Eagle Medium (DMEM) or the enriched Dulbecco's Modified Eagle Medium (eDMEM), RPMI, MEM, IMDM, opti-MEM medium and DMEM/F-12.

10. Use according any of claims 6 to 9, wherein the mass ratio aqueous solution / cell culture medium is comprised between 50:50 and 90:10, preferably between 60:40 and 80:20, more preferably the ratio is 70:30.

11. Use according to any of claims 6 to 10, wherein the gel further comprises cells, preferably the cells are selected from apical papilla stem cells, human bone marrow cells, human skin fibroblast, human gingival fibroblast, human umbilical vein cells, endothelial cells, hematopoietic stem cells (HSC), human induced pluripotent stem cells (HiPSC) and mesenchymal stem cells (MHSC).

12. Use according to any of the preceding claims, wherein the bioink formulation is suitable for bioprinting, preferably bioprinting is selected from comprising inkjet bioprinting, laser bioprinting and extrusion based bioprinting.

13. Bioprinting method comprising the use of a bioink formulation comprising a nucleolipid-based compound of formula I: 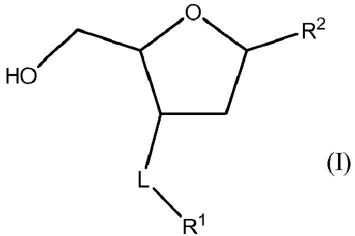 wherein,
- L represents an oxycarbonyl -O-C(O)- group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₂-C₃₀ hydrocarbon chain,
- R¹ represents
o a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether, or
o a diacylglycerol (or diglyceride) in which each acyl chain is a linear or branched C₂ to C₃₀ hydrocarbon chain, preferably C₆ to C₂₅ and more preferably C₈ to C₂₅, saturated or unsaturated, optionally completely or partially fluorinated, unsubstituted or substituted on the end of the chain by a fluorine atom or by a benzyl or naphtyl ester or ether,
- R² represents a purine or pyrimidine base selected from uracil, adenine, guanine, cytosine, thymine, hypoxanthine, or their derivatives, optionally substituted, or a non-natural mono- or bicyclic heterocyclic base each ring of which comprising form 4 to 7 members, preferably guanine, cytosine or thymine and more preferably thymine, and
- the compound is optionally in the form of a salt, preferably a metal cation salt and more preferably the metal cation may be selected from Li⁺, Na⁺, K⁺, Rb⁺, Cs⁺.

14. Nucleolipid-based compounds of formula I, II, III, IIIa, IIIb or IIIc for use in regenerative medicine, preferably for use in the bioprinting of vascular niches for cells, wherein the bioprinting is chosen in the group comprising inkjet bioprinting, laser bioprinting and extrusion based bioprinting.

15. Bioprinted grid or prototype construct comprising nucleolipid-based compounds of formula I, II, III, IIIa, IIIb or IIIc.

16. Bioink gel formulation, preferably a hydrogel or a supramolecular gel, comprising:
- between 0.1 and 10 % w/w of a compound of formula I, II, III, IIIa, IIIb or IIIc,
- a 0.5 to 1.5 wt. % % w/w aqueous lithium, sodium, potassium, rubidium or cesium solution,
- a cell culture medium, and
- optionally cells,
wherein the ratio aqueous solution / cell culture medium is comprised between 10:90 - 50:50.
